(19) Europäisches Patentamt
European Patent Office
Office européen des brevets

(11) Publication number: **0 517 412 A1**

# (12) EUROPEAN PATENT APPLICATION

(21) Application number: **92304738.5**

(51) Int. Cl.[5]: **A61K 31/55**, A61K 9/48

(22) Date of filing: **26.05.92**

(30) Priority: **03.06.91 GB 9111858**
**05.03.92 GB 9204774**

(43) Date of publication of application:
**09.12.92 Bulletin 92/50**

(84) Designated Contracting States:
**PT**

(71) Applicant: **MERCK SHARP & DOHME LTD.**
**Hertford Road**
**Hoddesdon Hertfordshire EN11 9BU(GB)**

(72) Inventor: **Lievens, Hildegard S.R.**
**5 Balfour Street**
**Hertford, Hertfordshire SG14 3AX(GB)**

(74) Representative: **Barrett-Major, Julie Diane et al**
**Merck & Co., Inc. European Patent Department Terlings Park Eastwick Road**
**Harlow Essex CM20 2OR(GB)**

## (54) Pharmaceutical formulations of a benzodiazepine.

(57) Pharmaceutical formulations comprising 3R(+)-N-(2,3-dihydro-1-methyl-2-oxo-5-phenyl-1H-1,4-benzodiazepin-3-yl)-N'-(3-methylphenyl)-urea and a pharmaceutically acceptable carrier, characterised in that the carrier comprises a pharmaceutically acceptable oil selected from esterification or polyether products of glycerides with vegetable oil fatty acids of chain length $C_8$-$C_{10}$ and a pharmaceutically acceptable surfactant selected from oleate and laurate esters of a polyalcohol copolymerised with ethylene oxide are disclosed. They optionally also contain a plasticiser and/or water and/or $\alpha$-D-tocopherol. Preferably, the formulations are prepared by known methods and are filled into hard or soft gelatin capsules. The formulations are useful for the treatment or prevention of medical conditions in which an excess of CCK is implicated such as pain, panic or anxiety.

EP 0 517 412 A1

The present invention relates to pharmaceutical formulations suitable for containing pharmacologically active agents with low water solubility. Particularly, the invention relates to benzodiazepine-containing formulations suitable for filling into soft gelatin capsules for oral administration.

One of the problems associated with oral administration of pharmacologically active agents with low water solubility is their resulting low bioavailability in vivo. Also, the distribution of the pharmacologically active agent in the gastrointestinal tract tends to be less homogeneous than desirable. This causes its bioavailability to be variable and not reliably reproducible.

One such pharmacoligically active ingredient is the benzodiazepine 3R(+)-N-(2,3-dihydro-1-methyl-2-oxo-5-phenyl-1H-1,4-benzodiazepin-3-yl)-N'-(3-methylphenyl)-urea of formula (A):

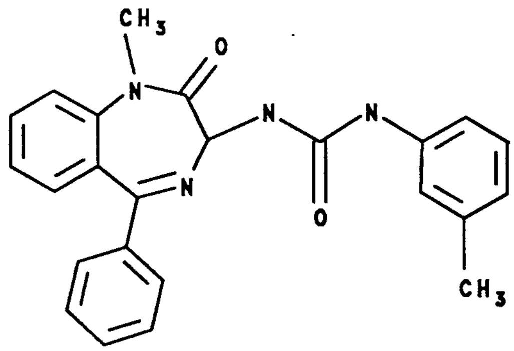

L-365,260

which has a solubility in water of less than 0.002mg/ml.

The benzodiazepine is a CCK antagonist and its synthesis and uses are described in, for example, European patent specifications numbers EPA-107,919, EPA-284,256 and EPA-434,364, which are herein incorporated by reference. In particular, the benzodiazepine and its pharmaceutical formulations are useful for the treatment or prevention of medical conditions in which an excess of CCK is implicated such as in treating panic, anxiety and analgesia.

It has now been found that pharmaceutical formulations wherein the composition is in the form of a self-emulsifying system have particularly advantageous properties in respect of the benzodiazepine (A).

A review on self-emulsifying systems by Colin W. Pouton can be found in International Journal of Pharmaceutics 27, 1985, 335-348. A self-emulsifying system is generally recognised to be a mixture of oil and surfactant which emulsifies in water under conditions of gentle agitation. Such mixtures may also be spontaneously emulsifying. Self-emulsifying formulations have been used by the herbicide and pesticide industries. Lipophilic herbicides are dissolved in organic solvents with surfactants to produce self-emulsifying concentrates which can be dispersed easily in local water prior to crop-spraying.

It has been suggested that self-emulsifying drug delivery systems can be prepared which, after oral administration in gelatin capsules, will emulsify within the gastric contents (Groves and de Galindez in Acta. Pharm. Suec. 13 (1976) 361-72). The mechanisms, rate and extent of drug absorption from the resulting emulsion will be strongly dependent on the particular oils and surfactants used in each formulation. For example, absorption will be influenced by whether or not the oil is digestible and by the partitioning of drug between the oil and water (Armstrong and James in Int. J. Pharm. 6 (1980), 195-204). Pouton found that self-emulsifying mixtures of excipients produced fine dispersions of droplets of diameter less than 1μm. However, Pouton looked only at mixtures of certain excipients in the absence of any pharmacologically active ingredient. Furthermore, he did not tackle the problem of finding a mixture suitable for adminstration in capsule form.

Self-emulsifying systems are a subclass or special type of emulsion, which spontaneously emulsify in water to form an oil-in-water emulsion, wherein the median diameter of the droplets is generally less than 2 microns. Usually, such systems have been referred to as microemulsions. Self-emulsifying systems are produced from an emulsifier (a surfactant) and a co-emulsifier (ie a co-surfactant, polar additive or co-solubilizer) which lowers the interfacial tension between the oil and water phases to typically less than 1 mN/m. Emulsions are well-known in other fields, eg cosmetic preparations, floor polishes, paints and foods. However, the formulation of emulsions and in particular microemulsions is to a certain extent largely empirical (see for example pp.34-56 in Microemulsions Theory and Practice, Ed. L. Prince, 1977).

J. Ziegenmeyer and C. Fuhrer in Acta Pharmaceutica Technologica 1980, 26 (4) pp.273-275 have disclosed a microemulsion pharmaceutical composition containing 1% tetracycline hydrochloride and

decanol. However, the composition is not capable of producing a systemic therapeutic effect as the tetracycline concentration in the pharmaceutical composition is too low. More importantly, decanol is not pharmaceutically acceptable.

Microemulsions have been suggested for topical administration such as in British patent specification number 2098865 which discloses a skin penetrating pharmaceutical composition incorporating a skin penetrable pharmacologically active agent devoid of hydroxyl groups wherein the composition is in the form of a stable microemulsion formed from skin compatible excipients. Such microemulsions may be produced in conventional manner for the preparation of topical pharmaceutical compositions. The skin compatible pharmacologically active agent (0.01-15%), water-immiscible organic solvent (5-30%), water (15-55%), emulsifier (10-30%) and co-emulsifier (4-30%) may be heated together and the mixture cooled.

Therefore, British patent specification number 2098865 still does not solve the problem of how to incorporate an active ingredient into a self-emulsifying capsule formulation for oral administration. Furthermore, the problem of obtaining a formulation having good solubilising power for the low-water soluble benzodiazepine is not solved thereby.

In contrast, the present invention relates to a formulation which is suitable for oral administration and which provides the pharmacologically active ingredient in solution in a pharmaceutically acceptable carrier, which formulation is self-emulsifying in an aqueous medium.

An oral formulation is known from British patent specification no. 2015339 which contains cyclosporin A as pharmacologically active ingredient and a carrier comprising Labrafil M1944CS and ethanol (40:15), and corn oil or olive oil in proportions 3:10:3:5, respectively, in the form of a drink solution. There is also disclosed, as alternative carrier, a mixture of glycerol mono-oleate and ethanol, which formulation can be filled into soft gelatin capsules. However, when the low-water soluble benzodiazepine substitutes the cyclosporin in this type of formulation, its solubility is still low in the region of 20 mg/g.

Belgian patent specification No. 895 724 also discloses soft gelatin capsules containing dihydrocyclosporin D as pharmacologically active ingredient (15-25%) and a carrier comprising ethanol (2-5%), Imwitor 742 (10-40%) and an esterified corn oil (40-60%). When the low-water soluble benzodiazepine substitutes the dihydrocyclosporin in this type of formulation, its solubility is in the region of 60 mg/g. However, the formulation still contains ethanol which it is preferred to avoid. These types of formulation therefore still do not solve the problem of how to obtain a formulation having good solubilising power for the low-water soluble benzodiazepine.

British patent specification no. GB 2 228 198 also relates to certain cyclosporin-containing compositions wherein the carrier comprises (b) a fatty acid triglyceride; (c) a glycerol fatty acid partial ester or propylene glycol or sorbitol complete or partial ester; and (d) a tenside (HLB≧10). Therein are disclosed specific examples wherein the carrier comprises (b) Miglyol 812, (c) Imwitor 742 and (d) Cremophore RH40 or wherein the carrier comprises (b) Estasan GT8-60, (c) Arlacel 186 and (d) Tween 80. These compositions are oil-based, are not agueous emulsions and do not include ethanol or Labrafils or the like. However, these require two components (b) and (c) in addition to a tenside. There is also disclosed one example wherein the carrier comprises (b) + (c) Maisine and (d) Cremophore RH40. Maisine is based on long chain ($C_{16}$ - $C_{18}$) fatty acids. When the benzodiazepine is substituted for the cyclosporin in this type of formulation, the carrier is again found to be a poor solvent therefor in the region of less than 10mg/g.

We have surprisingly found that if a medium chain length ($C_8$-$C_{10}$) fatty acid glyceride optionally esterified with polyethylene glycol is chosen for the oily component and an oleate or laurate ester of a polyoxyethylene polyalcohol is chosen as a surfactant, then satisfactory formulations (wherein the benzodiazepine is soluble in the carrier) can be prepared.

Thus, for example when Imwitor 742 is employed as the oil in the formulations of the present invention it is not necessary also to include, for example, Miglyol 812 as a second oily component. We also find that use of Labrafils in the formulations of the present invention does not require the presence of ethanol or other volatile solvents.

Therefore, the present invention provides a pharmaceutical formulation suitable for filling into soft gelatin capsules which formulation comprises 3R(+)-N-(2,3-dihydro-1-methyl-2-oxo-5-phenyl-1H-1,4-benzodiazepin-3-yl)-N'-(3-methylphenyl)-urea of formula (A) and a pharmaceutically acceptable carrier, characterised in that the carrier comprises a pharmaceutically acceptable oil selected from esterification or polyether products of glycerides with vegetable oil fatty acids of chain length $C_8$-$C_{10}$, a pharmaceutically acceptable surfactant selected from oleate and laurate esters of a polyalcohol copolymerised with ethylene oxide, and optionally a plasticiser and/or water in which carrier the active ingredient is soluble such that the formulation is self-emulsifying in an aqueous medium.

The solubility of the benzodiazepine in the mixture of ingredients of the carrier is surprisingly found to be more than in the separate ingredients.

Preferably, the carrier comprises a pharmaceutically acceptable oil selected from the class consisting of liquid or highly viscous medium-chain glycerides optionally esterified with polyethylene glycol and a pharmaceutically acceptable surfactant which is liquid in association with the oil, in which carrier the active ingredient is soluble such that the formulation is self-emulsifying in an aqueous medium. Such carriers eliminate the need for including a plasticiser and/or water. Preferred carriers of the formulations of the present invention have a maximum solubility for the benzodiazepine in the region of 140mg/g (comprising Imwitor 742 and Tween 80) to 240mg/g (Labrasol/Tween 80). However, even the corresponding Labrafil-containing carrier of the formulation of the present invention has a maximum solubility for the benzodiazepine in the region of 60-80mg/g.

Preferably, the pharmaceutical formulation is in the form of soft gelatin capsules which, when dropped into water (or when in contact with the gastro-intestinal tract) break open and form an emulsion without stirring. The median diameter of the droplets in such self-emulsifying systems are typically less than two microns. However, hard gelatin capsules may also be used.

The oil, surfactant and any plasticiser are preferably different ingredients although an individual ingredient may perform more than one function. The oil, surfactant and any plasticiser are chosen so that the active ingredient is adequately soluble in a mixture thereof, and so that the release rate of the active ingredient is pharmacologically acceptable.

The formulations according to the present invention may contain:

pharmaceutically acceptable oil 38.5-82%
pharmaceutically acceptable surfactant 15-70%
plasticiser 0 to 5%
purified water 0.01 to 7%
active ingredient 0.2 to 80%

subject to the need for the active ingredient to be soluble and for the formulation to be self-emulsifying in an aqueous system.

For example, in formulations where a plasticiser is required, the formulations according to the present invention contain:

pharmaceutically acceptable oil 50-82%
pharmaceutically acceptable surfactant 15-30%
plasticiser 3 to 5%
purified water 0.01% to 7%
active ingredient 0.2 to 80%

However, the preferred formulations according to the present invention contain:

pharmaceutically acceptable oil 38.5-70%
pharmaceutically acceptable surfactant 30-70%
plasticiser 0 to 3%
purified water 0% to 3%
active ingredient 0.2 to 14%

In the above formulations, the preferred range of active ingredient is 0.2 to 30%.

The ratio of oil:surfactant in the preferred formulations is preferably in the range of from 70:30 to 30:70. If the surfactant comprises greater than about 70%, its emulsifying characteristics and solubilising power reduce; and significantly below 30%, the formulation loses its self-emulsifying characteristics.

Therefore, the pharmaceutically acceptable oil is the main solvent for the active ingredient. Such solvents are liquid or highly viscous, becoming liquid in association with the surfactant, and are esterification or polyether products of glycerides with vegetable oil fatty acids. These include glycolised ethoxylated glycerides such as: polyethoxylated glycerides esterified with fatty acids of a vegetable oil such as corn oil or apricot kernel oil, in particular polyethoxylated oleic linoleic glycerides such as Labrafil M2125 CS (trade mark), esters of glycerides with apricot kernel oil such as Labrafil M1944CS (trade mark), saturated polyglycolised $C_8$-$C_{10}$ glycerides such as esters of glycerides with caprylic/capric acid in particular with PEG 8 caprylate/caprate such as Labrasol (trade mark), partial glycerides of medium-chain vegetable fatty acids such as Imwitor 742 (trade mark), and other caprylic/capric glycerides such as Imwitor 908 (trade mark). Especially preferred are Labrafil M1944CS or Labrafil M2125 CS, Labrasol, particularly Imwitor 742 and Imwitor 908.

The pharmaceutically acceptable surfactant is liquid in association with the oil and is an oleate or laurate ester of a polyalcohol copolymerised with ethylene oxide such as in particular a polyoxyethylene (20) sorbitan mono-oleate such as Tween 80 (trade mark), a laurate ester such as polyoxyethylene (20) sorbitan mono-laurate such as Tween 20 (trade mark) or a polyoxyethylene glycerol trioleate. The surfactant is preferably Tween 80 or Tween 20, more preferably Tween 80.

Any plasticiser present is to prevent break-up of any soft gelatin capsule shell into which the formulation is to be filled, but must be chosen so that it does not affect adversely the self-emulsifying nature of the formulations. Preferred plasticisers are propylene glycol and sorbitol, especially propylene glycol. However, using, for example, an Imwitor/Tween carrier renders the use of a plasticiser unnecessary.

Additional ingredients, such as an antioxidant such as α-D-tocopherol(vitamin E) may optionally be present. In the case of an Imwitor-containing carrier, vitamin E is preferably incorporated to about 1%.

Therefore, a preferred formulation comprises:
esterification or polyether product of triglycerides with vegetable oil fatty acids: 38.5 to 82%
oleate ester of a polyalcohol copolymerised with ethylene oxide: 15 to 70%
plasticiser: 0 to 5%
purified water: 0 to 7%
(A): up to 14%
α-D-tocopherol: about 1%.

When the carrier is other than an Imwitor/Tween combination, then it preferably comprises:
esterification or polyether product of triglycerides with vegetable oil fatty acids: 50 to 82%
oleate ester of a polyalcohol copolymerised with ethylene oxide: 15 to 30%
plasticiser: 3 to 5%
purified water: 0.01 to 7%
(A): up to 8%.

Thus, a preferred formulation according to the present invention contains:
Labrafil: 50% to 82%
Tween 80: 15 to 30%
propylene glycol: 3 to 5%
purified water: 0.01% to 7%
(A): 0.2 to 80%
Otherwise, it preferably comprises:
glycerides (caprilic/capric): 38.5% to 70%
polyoxyethylated (20) sorbitan ester: 30 to 70%
plasticiser: 0 to 3%
purified water: 0 to 3%
(A): up to 14%
α-D-tocopherol: about 1%
A particularly preferred formulation according to the present invention contains:
Imwitor 742 or 908 or Labrasol: 38.5 to 70%
Tween 80 or 20: 30 to 70%
propylene glycol: 0 to 3%
purified water: 0 to 3%
(A): 0.2 to 14%
α-D-tocopherol: about 1%.

Formulations according to the present invention may be prepared by methods known to those skilled in the art for forming self-emulsifying systems, and involve bringing the ingredients into intimate physical admixture.

For example, the ingredients for the pharmaceutically acceptable carrier optionally except the water may be mixed, heated (for example, to a temperature in the range of from 60 to 80 ° C, preferably from about 65 to 75 ° C, such as about 70 ° C), the water added (if not already present) followed by cooling of the mixture to room temperature. At this point, the active ingredient can be added and the mixture stirred followed by heating to ensure that the drug is in solution in the vehicle (for example, from 40 to 70 ° C, preferably around 50 ° c). After cooling, the mixture may then be filled into soft gelatin capsules.

Although heating during the process of preparing the formulations according to this invention is preferable, it is not necessary for formulations containing up to around 60mg/g active ingredient or unless the oil is waxy. For example, the oil may be heated as explained above until clear and cooled before addition of the other carrier ingredients. However, without heating, a lower percentage of the active ingredient can be dissolved in the carrier, for example, around 6% as opposed to around 7 to 8% when heating. On using the room temperature method, in order to obtain a clear homogeneous solution, a Silversen mixer or another high shear type mixer is preferably used to overcome the molecular adhesion forces.

The formulations may then be filled into hard or soft gelatin capsules, as desired. Preferably, these are then presented in blister packs, for example, in aluminium foil sheets having PVC 'blisters'. Conveniently,

28 capsules may be presented in each sheet, and each pack may contain four such sheets. The pack conveniently contains a pack insert comprising instructions for the use of the capsules for the treatment or prevention of medical conditions in which an excess of CCK is implicated such as in treating panic, anxiety and analgesia.

The present invention will now be illustrated with reference to the following examples:

Method 1 (Heating method)

LABRAFIL M2125 CS, TWEEN 80 and propylene glycol are weighed and poured into a tared stainless steel vessel and mixed using a spatula. The active ingredient (drug) is added to the liquid and further stirred until all the drug is mixed into the liquid. This mix is heated on a hot plate to 70 °C until the drug is dissolved. During heating time, the liquid is stirred continuously using a spatula. Once a clear solution is obtained, the vessel is taken from the hot plate and the required amount of purified water is added while the solution is still warm. The solution becomes hazy as soon as the water is added, but turns clear after further stirring and stays clear after cooling down.
Using this method the maximum solubility for benzodiazepine (A) is about 7% to 8% (w/w).

Method 2 (Room temperature method)

LABRAFIL M2125 CS, TWEEN 80, propylene glycol and purified water are poured into a suitable vessel. The temperature is 20 °C (ie room temperature). The mixer head of a Silverson mixer is lowered down near the bottom of the vessel to avoid air entrapment in the liquid. The liquid is mixed until homogeneous. The active ingredient (drug) is added and stirred into the liquid using a low mixing speed, which is then gradually increased to an average mixing speed of 4000 to 5000 rpm until the drug is dissolved.
A clear solution is obtained when all the air is removed out of the liquid. Using this method up to about 6% (w/w) of benzodiazepine (A) can be dissolved.

Method 3

IMWITOR is heated at 40-50 °C to melt the solid phase which may appear at room temperature. After cooling, the IMWITOR is mixed with TWEEN and any other ingredients using a high shear type mixer such as a Silversen mixer. The active ingredient is dissolved in this liquid by further mixing. Once all dissolved, air can be removed using an ultrasonic bath.

Examples 1 to 4: Formulations for use in methods 1 and 2

Example 1

The formula of the carrier is as follows:

| Ingredients | Quantity |
|---|---|
| Labrafil M2125CS or M1944CS | 62% |
| Tween 80 | 30% |
| propylene glycol | 3% |
| purified water | 5% |

Benzodiazepine (A) is dissolved in this carrier at a concentration in the range of from 0.2 to 80mg/g, for example 60mg/g

Example 2

| Ingredients | Quantity |
|---|---|
| Labrafil M2125 CS | 71% |
| Tween 80 | 15% |
| purified water | 5% |
| propylene glycol | 3% |
| (A) | 6% |

Example 3

| Ingredients | Quantity |
|---|---|
| Labrafil M1944 CS | 70% |
| Tween 80 | 20% |
| purified water | 1% |
| propylene glycol | 3% |
| (A) | 6% |

Example 4

| Ingredients | Quantity |
|---|---|
| Labrafil M2125 CS | 46% |
| Tween 80 | 30% |
| propylene glycol | 3% |
| purified water | 5% |
| Ethanol | 10% |
| (A) | 6% |

Examples 5 to 8: Formulations for use in method 3

Example 5

| Ingredients | Quantity |
|---|---|
| Imwitor 908 | 42.5% |
| Tween 80 | 42.5% |
| α-D-Tocopherol | 1% |
| (A) | 14% |

Example 6

| Ingredients | Quantity |
|---|---|
| Imwitor 742 | 39.5% |
| Tween 80 | 42.5% |
| propylene glycol | 3% |
| Purified water | 3% |
| α-D-Tocopherol | 1% |
| (A) | 14% |

Example 7

| Ingredients | Quantity |
|---|---|
| Imwitor 742 | 42.5% |
| Tween 20 | 42.5% |
| α-D-Tocopherol | 1% |
| (A) | 14% |

Example 8

| Ingredients | Quantity |
|---|---|
| Imwitor 742 | 256mg |
| Tween 20 | 256mg |
| α-D-Tocopherol | 6mg |
| (A) | 70mg |

Example 9: Capsules

The formulations of examples 1 to 8 are filled into hard or soft gelatin capsules.

Example 10: Blister packs

The capsules of example 9 are packaged in blister packs of aluminium foil/PVC consisting of 28 capsules per sheet and 4 such sheets per pack. Included in the pack is the pack insert.

**Claims**

**1.** A pharmaceutical formulation comprising 3R(+)-N-(2,3-dihydro-1-methyl-2-oxo-5-phenyl-1H-1,4-benzodiazepin-3-yl)-N'-(3-methylphenyl)-urea of formula (A):

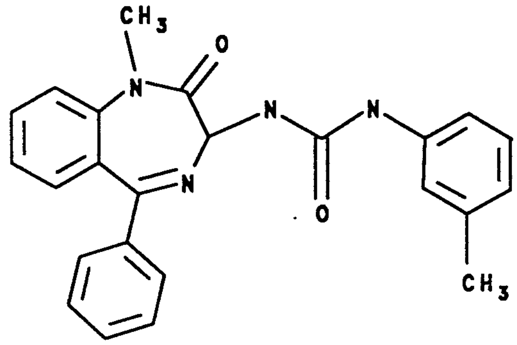

L-365,260

and a pharmaceutically acceptable carrier, characterised in that the carrier comprises a pharmaceutically acceptable oil selected from esterification or polyether products of glycerides with vegetable oil fatty acids of chain length $C_8$-$C_{10}$ and a pharmaceutically acceptable surfactant selected from oleate and laurate esters of a polyalcohol copolymerised with ethylene oxide.

**2.** A formulation according to claim 1 wherein the carrier comprises a pharmaceutically acceptable oil selected from the class consisting of liquid or highly viscous medium-chain glycerides optionally esterified with polyethylene glycol and a pharmaceutically acceptable surfactant which is liquid in association with the oil.

**3.** A formulation according to claim 1 or claim 2 wherein the carrier further comprises one or more further ingredients selected from a plasticiser, water and α-D-tocopherol.

**4.** A formulation according to any preceding claim comprising:
pharmaceutically acceptable oil 50-82%
pharmaceutically acceptable surfactant 15-30%
plasticiser 3 to 5%
purified water 0.01 to 7%
active ingredient 0.2 to 80%.

**5.** A formulation according to any preceding claim comprising:
pharmaceutically acceptable oil 38.5-70%
pharmaceutically acceptable surfactant 30-70%
plasticiser 0 to 3%
purified water 0% to 3%
active ingredient 0.2 to 14%

**6.** A formulation according to any preceding claim wherein the pharmaceutically acceptable oil is selected from Labrafil M1944 CS, Labrafil M2125 CS, Labrasol, Imwitor 742 and Imwitor 908.

**7.** A formulation according to any preceding claim wherein the pharmaceutically acceptable surfactant is selected from Tween 80 and Tween 20.

**8.** A formulation according to any preceding claim comprising 3R(+)-N-(2,3-dihydro-1-methyl-2-oxo-5-phenyl-1H-1,4-benzodiazepin-3-yl)-N'-(3-methylphenyl)-urea (70mg), Imwitor 742 (256mg), Tween 80 (256mg) and α-D-tocopherol (6mg).

**9.** A formulation according to any preceding claim for use in the treatment or prevention of pain, panic or anxiety.

**10.** A gelatin capsule containing a formulation according to any preceding claim.

**11.** A method for the preparation of a formulation according to any preceding claim comprising bringing the

ingredients into intimate physical admixture.

**12.** A method for the preparation of a gelatin capsule according to claim 10 comprising preparing a formulation according to claim 11 and filling it into a hard or soft gelatin capsule.

**13.** A blister pack comprising a plurality of capsules according to claim 10 in association with instructions for use of the capsules in the treatment or prevention of pain, panic or anxiety.

European Patent Office

# EUROPEAN SEARCH REPORT

Application Number

EP 92 30 4738

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 411 668 (WARNER LAMBERT COMPANY)<br>* page 8, line 44 - line 52 *<br>* page 9, line 19 - line 23 *<br>* page 9, line 43 - line 49 *<br>* page 11; example 3 *<br>--- | 1 | A61K31/55<br>A61K9/48 |
| A | FR-A-2 372 635 (R.P. SCHERER LIMITED)<br>* page 2, line 13 - line 16 *<br>* page 3, line 8 - page 4, line 15 *<br>* page 8; example 1 *<br>* claims 1,5,7,8 *<br>--- | 1,2 | |
| A | GB-A-1 600 639 (KALI CHEMIE PHARMA GMBH)<br>* page 5; example 6 *<br>--- | 1,2 | |
| A | EP-A-0 225 189 (R.P. SCHERER CORPORATION)<br>* page 15 - page 16; examples 4-9 *<br>--- | 1,2 | |
| A | CHEMICAL ABSTRACTS, vol. 90, no. 10, 5 March 1979, Columbus, Ohio, US;<br>abstract no. 76504W,<br>BOBBE D. ET AL: 'Effect of some excipients and adjuvants on the dissolution rate of amidopyrine present in soft capsules'<br>page 280 ;column 1 ;<br>* abstract *<br>----- | 1,2 | TECHNICAL FIELDS SEARCHED (Int. Cl.5)<br>A61K |

The present search report has been drawn up for all claims

| Place of search | Date of completion of the search | Examiner |
|---|---|---|
| THE HAGUE | 11 AUGUST 1992 | BOULOIS D. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)